(19) Europäisches Patentamt
European Patent Office
Office européen des brevets

(11) Veröffentlichungsnummer: **0 196 583 B1**

# (12) EUROPÄISCHE PATENTSCHRIFT

(45) Veröffentlichungstag der Patentschrift: **23.10.91**

(51) Int. Cl.$^5$: **A61K 31/41**, A61K 31/415

(21) Anmeldenummer: **86103970.9**

(22) Anmeldetag: **22.03.86**

## (54) Verwendung von Azolylmethyloxiranen zur Bekämpfung von viralen Erkrankungen.

(30) Priorität: **29.03.85 DE 3511411**

(43) Veröffentlichungstag der Anmeldung:
**08.10.86 Patentblatt 86/41**

(45) Bekanntmachung des Hinweises auf die Patenterteilung:
**23.10.91 Patentblatt 91/43**

(84) Benannte Vertragsstaaten:
**AT BE CH DE FR GB IT LI NL SE**

(56) Entgegenhaltungen:
**EP-A- 0 094 564**
**EP-A- 0 132 508**
**FR-A- 2 534 808**
**US-A- 3 952 103**
**US-A- 4 438 129**

(73) Patentinhaber: **BASF Aktiengesellschaft**
**Carl-Bosch-Strasse 38**
**W-6700 Ludwigshafen(DE)**

(72) Erfinder: **Janssen, Bernd, Dr.**
**Leuschnerstrasse 18a**
**W-6700 Ludwigshafen(DE)**
Erfinder: **Karbach, Stefan, Dr.**
**Sperlinggasse 3**
**W-6700 Ludwigshafen(DE)**
Erfinder: **Meyer, Norbert, Dr.**
**Dossenheimer Weg 22**
**W-6802 Ladenburg(DE)**
Erfinder: **Lauer, Gerhard, Dr.**
**Stephanienufer 19**
**W-6800 Mannheim(DE)**

EP 0 196 583 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Es ist bereits bekannt, daß Azolderivate wie z.B. 2-(α-Hydroxybenzyl)-benzimidazol (vgl. Burger's Medicinal Chemistry, IV. Ausg. 1979, Teil 2, S. 554 ff) und Hydroxyethylazole, wie z.B. 2-(4-Chlorphenoxymethyl)-3,3-dimethyl-1-(1,2,4-triazol-1-yl)-2-butanol (= Vibunazol, DE-OS 32 38 903) aber auch andere Vertreter dieser Stoffklasse (DE-OS 33 15 808), antivirale Wirksamkeit entfalten. Azolylmethyloxirane der Formel I

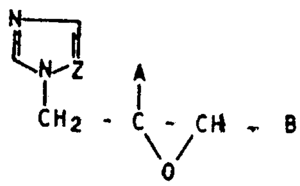

(I),

in der

A und B gleich oder verschieden sind und Alkyl mit 1 bis 4 Kohlenstoffatomen, Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen, Phenoxy sowie den Phenylsulfonylrest substituiert sein kann,

Z für eine CH-Gruppe oder ein Stickstoffatom steht

sowie deren physiologisch verträgliche Säureadditionssalze sind bereits als lokal und systemisch hoch wirksame Antimykotika bekannt (DE-OS 32 18 129).

Es wurde nun überraschenderweise gefunden, daß die oben genannten Azolylmethyloxirane der Formel I auch eine sehr gute antivirale Wirkung besitzen.

Die Verbindungen der Formel I enthalten chirale Zentren und werden im allgemeinen in Form von Racematen oder als Diastereomerengemische von erythro- sowie threo-Formen enthalten. Die erythro- und threo-Diastereomeren lassen sich bei den erfindungsgemäßen Verbindungen beispielsweise aufgrund ihrer unterschiedlichen Löslichkeit oder durch Säulenchromatographie trennen und in reiner Form isolieren. Aus solchen einheitlichen Diastereomerenpaaren kann man mit bekannten Methoden einheitliche Enantiomere erhalten. Sowohl die einheitlichen Diastereomeren bzw. Enantiomeren als auch deren bei der Synthese anfallende Gemische können als antivirale Wirkstoffe verwendet werden.

Als Beispiele für Verbindungen der Formel I seien speziell folgende genannt:

| Nr. | A | B | Z | Diastereomer | Fp. [°C] |
|---|---|---|---|---|---|
| 1 | 4-Cl-$C_6H_4$ | 2,4-$Cl_2$-$C_6H_3$ | CH | A | 102-103 |
| 2 | 4-Cl-$C_6H_4$ | 2,4-$Cl_2$-$C_6H_3$ | CH | B | 109 |
| 3 | 4-Cl-$C_6H_4$ | 2,4-$Cl_2$-$C_6H_3$ | N | A | 119 |
| 4 | 4-Br-$C_6H_4$ | $C_6H_5$ | CH | A | 152-153 |
| 5 | 4-Br-$C_6H_4$ | 4-Cl-$C_6H_4$ | CH | A | 143-144 |
| 6 | $C_6H_5$ | 2,4-$Cl_2$-$C_6H_3$ | CH | A | 103 |
| 7 | 4-Br-$C_6H_4$ | 2,4-$Cl_2$-$C_6H_3$ | CH | A | 107-108 |
| 8 | 4-,4-$Cl_2$-$C_6H_3$ | 4-Cl-$C_6H_4$ | CH | A | 135 |
| 9 | 4-Cl-$C_6H_4$ | $C_6H_5$ | CH | A | 138 |
| 10 | 4-Br-$C_6H_4$ | 2,4-$Cl_2$-$C_6H_3$ | N | A | 133-134 |
| 11 | $CH_3$ | 2,4-$Cl_2$-$C_6H_3$ | CH | B | 113-117,5 |
| 12 | $CH_3$ | 2,4-$Cl_2$-$C_6H_3$ | CH | A | 98-104 |
| 13 | $C(CH_3)_3$ | 4-Cl-$C_6H_4$ | N | A | 79- 80 |
| 14 | $C(CH_3)_3$ | 4-Cl-$C_6H_4$ | CH | A x HCl | 214-216 |
| 15 | $C(CH_3)_3$ | $C_6H_5$ | N | A x HCl | 148 |
| 16 | $C(CH_3)_3$ | $C_6H_5$ | CH | A | 75 |
| 17 | $C(CH_3)_3$ | 2,4-$Cl_2$-$C_6H_3$ | N | A | 124 |
| 18 | $C(CH_3)_3$ | 2,4-$Cl_2$-$C_6H_3$ | CH | A | 95 |
| 19 | 4-Cl-$C_6H_4$ | 4-$C(CH_3)_3$-$C_6H_4$ | CH | B | 160-162 |
| 20 | 4-Cl-$C_6H_4$ | 4-$C(CH_3)_3$-$C_6H_4$ | N | A | 176-177 |
| 21 | 2,4-$Cl_2$-$C_6H_3$ | 4-$C(CH_3)_3$-$C_6H_4$ | CH | A | 132-134 |
| 22 | $CH_3$ | 2,4-$Cl_2$-$C_6H_3$ | N | A | 105-108 |
| 23 | $CH_3$ | 2,4-$Cl_2$-$C_6H_3$ | N | B | 80- 85 |
| 24 | $CH_3$ | 2,4-$Cl_2$-$C_6H_3$ | N | A : B = 1 : 1 | 70- 81 |

| Nr. | A | B | Z | Diastereomer | Fp. [°C] |
|---|---|---|---|---|---|
| 25 | 4-$C(CH_3)_3$-$C_6H_4$ | 4-Cl-$C_6H_4$ | CH | A | 100-152 |
| 26 | 4-$C(CH_3)_3$-$C_6H_4$ | 4-Cl-$C_6H_4$ | N | A | 105-107 |
| 27 | 4-$C(CH_3)_3$-$C_6H_4$ | 2,4-$Cl_2$-$C_6H_3$ | CH | A | 101-113 |
| 28 | 4-$C(CH_3)_3$-$C_6H_4$ | 2,4-$Cl_2$-$C_6H_3$ | N | A | 108-111 |
| 29 | 4-Cl-$C_6H_4$ | 3-$OCH_3$-$C_6H_4$ | CH | A x HCl | 173 |
| 30 | 4-Cl-$C_6H_4$ | 3-$OCH_3$-$C_6H_4$ | N | A | 77 |
| 31 | $C_6H_5$ | 2,4-$Cl_2$-$C_6H_3$ | N | A | 159-161 |
| 32 | 4-Cl-$C_6H_4$ | 3-$CF_3$-$C_6H_4$ | CH | A | 101-104 |
| 33 | 4-Cl-$C_6H_4$ | 3-$CF_3$-$C_6H_4$ | N | A | 107-109 |
| 34 | $C_6H_5$ | 3-$CF_3$-$C_6H_4$ | CH | A | 77- 78,5 |
| 35 | $C_6H_5$ | 3-$CF_3$-$C_6H_4$ | N | A x HCl | 131-133 |
| 36 | $C_6H_5$ | $C_6H_5$ | CH | A | 108-110 |
| 37 | 4-Cl-$C_6H_4$ | 4-F-$C_6H_4$ | CH | A | 130-132 |
| 38 | $C_6H_5$ | 4-Cl-$C_6H_4$ | CH | A | 105-106 |
| 39 | $C_6H_5$ | $C_6H_5$ | N | A | 116-118 |
| 40 | $C_6H_5$ | 4-Cl-$C_6H_4$ | N | A | 114-115 |
| 41 | 4-Cl-$C_6H_4$ | $C_6H_5$ | N | A | 106-110 |
| 42 | 4-$C_6H_5$-$C_6H_4$ | 4-Cl-$C_6H_4$ | N | A | 163-165 |
| 43 | 4-Br-$C_6H_4$ | $C_6H_5$ | N | A | 115-120 |
| 44 | 4-Br-$C_6H_4$ | 4-Cl-$C_6H_4$ | N | A | 115-120 |
| 45 | 4-Cl-$C_6H_4$ | 4-F-$C_6H_4$ | N | A | 112-117 |
| 46 | 4-Cl-$C_6H_4$ | 4-Br-$C_6H_4$ | N | A | 115-119 |
| 47 | 4-Cl-$C_6H_4$ | 4-Br-$C_6H_4$ | CH | A | 114-116 |
| 48 | 4-Cl-$C_6H_4$ | 4-Br-$C_6H_4$ | CH | B | 179-181 |

| Nr. | A | B | Z | Diastereomer | Fp. [°C] |
|---|---|---|---|---|---|
| 49 | $2,4-Cl_2-C_6H_3$ | $4-Br-C_6H_4$ | CH | A | 135-139 |
| 50 | $4-Cl-C_6H_4$ | $4-F-C_6H_4$ | N | B | 219-223 |
| 51 | $4-Cl-C_6H_4$ | $4-Br-C_6H_4$ | N | B | 210-213 |
| 52 | $2,4-Cl_2-C_6H_3$ | $4-Br-C_6H_4$ | N | A | 108-110 |
| 53 | $2,4-Cl_2-C_6H_3$ | $C_6H_5$ | CH | A | Harz |
| 54 | $2,4-Cl_2-C_6H_3$ | $C_6H_5$ | CH | B | 118-121 |
| 55 | $2,4-Cl_2-C_6H_3$ | $C_6H_5$ | N | A | Harz |
| 56 | $2,4-Cl_2-C_6H_3$ | $C_6H_5$ | N | B | Harz |
| 57 | $4-(SO_2-C_6H_5)-C_6H_4$ | $4-Cl-C_6H_4$ | CH | A | 193-195 |
| 58 | $4-(SO_2-C_6H_5)-C_6H_4$ | $4-Cl-C_6H_4$ | CH | B | 204-205 |
| 59 | $4-(SO_2-C_6H_5)-C_6H_4$ | $4-Cl-C_6H_4$ | N | A | 132-135 |
| 60 | $4-(SO_2-C_6H_5)-C_6H_4$ | $4-Cl-C_6H_4$ | N | B | 175-177 |
| 61 | $2,4-Cl_2-C_6H_3$ | $4-Cl-C_6H_4$ | N | A | |
| 62 | $4-C_6H_5-C_6H_4$ | $4-Cl-C_6H_4$ | CH | | |
| 63 | $2-Cl-C_6H_4$ | $4-Cl-C_6H_4$ | CH | | |
| 64 | $2-Cl-C_6H_4$ | $4-Cl-C_6H_4$ | N | | |
| 65 | $4-Cl-C_6H_4$ | $3-Cl-C_6H_4$ | CH | | |
| 66 | $4-Cl-C_6H_4$ | $3-Cl-C_6H_4$ | N | | |
| 67 | $C_6H_5$ | $3,4-Cl-C_6H_3$ | CH | B | 103-105 |
| 68 | $C_6H_5$ | $3,4-Cl-C_6H_3$ | N | A | Harz |
| 69 | $3,5-Cl_2-C_6H_3$ | $4-Cl-C_6H_4$ | CH | | |
| 70 | $3,5-Cl_2-C_6H_3$ | $4-Cl-C_6H_4$ | N | | |
| 71 | $2-OCH_3-C_6H_4$ | $2,4-Cl_2-C_6H_3$ | CH | | |
| 72 | $2-OCH_3-C_6H_4$ | $2,4-Cl_2-C_6H_3$ | N | | |

| Nr. | A | B | Z | Diastereomer | Fp. [°C] |
|---|---|---|---|---|---|
| 73 | 4-O-C($CH_3$)$_3$-$C_6H_4$ | 2,4-$Cl_2$-$C_6H_3$ | CH | | |
| 74 | 4-O-C($CH_3$)$_3$-$C_6H_4$ | 2,4-$Cl_2$-$C_6H_3$ | N | | |
| 75 | 4-$CH_3$-$C_6H_4$ | $C_6H_5$ | CH | | |
| 76 | 4-$CH_3$-$C_6H_4$ | $C_6H_5$ | N | | |
| 77 | 4-(O-$C_6H_5$)-$C_6H_4$ | 4-Br-$C_6H_4$ | CH | | |
| 78 | 4-(O-$C_6H_5$)-$C_6H_4$ | 4-Br-$C_6H_4$ | N | | |
| 79 | $C_6H_5$ | 4-$NO_2$-$C_6H_4$ | CH | | |
| 80 | $C_6H_5$ | 4-$NO_2$-$C_6H_4$ | N | | |
| 81 | 2-$C_{10}H_7$ | 2,4-$Cl_2$-$C_6H_3$ | CH | A | 135 |
| 82 | 2-$C_{10}H_7$ | 2,4-$Cl_2$-$C_6H_3$ | N | A | 151 |
| 83 | 4-Cl-$C_6H_4$ | 1-$C_{10}H_7$ | CH | | |
| 84 | 4-Cl-$C_6H_4$ | 1-$C_{10}H_7$ | N | | |
| 85 | 4-Cl-$C_6H_4$ | 4-Cl-$C_6H_4$ | CH | A | 115-120 |
| 86 | 4-Cl-$C_6H_4$ | 4-Cl-$C_6H_4$ | N | A | 105-108 |
| 87 | 2-$C_{10}H_7$ | 4-Cl-$C_6H_4$ | CH | A | 140 |
| 88 | 2-$C_{10}H_7$ | 4-Cl-$C_6H_4$ | N | A | 107 |
| 89 | 2,4-$Cl_2$-$C_6H_3$ | 4-C($CH_3$)$_3$-$C_6H_4$ | CH | B | 142-143 |
| 90 | 3,4-$Cl_2$-$C_6H_3$ | 4-Br-$C_6H_4$ | N | A | 130-134 |
| 91 | 4-Br-$C_6H_4$ | 4-F-$C_6H_4$ | N | A | 137 |
| 92 | 4-Br-$C_6H_4$ | 4-F-$C_6H_4$ | N | B | Öl |
| 93 | 4-Br-$C_6H_4$ | 4-F-$C_6H_4$ | CH | A | 152 |
| 94 | $C_6H_5$ | 2-Cl-$C_6H_4$ | N | A | 158-159 |
| 95 | 4-Cl-$C_6H_4$ | 2-Cl-$C_6H_4$ | N | A | 166 |
| 96 | $C_6H_5$ | 2-Cl-$C_6H_4$ | CH | A/B = 65 : 35 | 85- 87 |
| 97 | 4-Cl-$C_6H_4$ | 2-Cl-$C_6H_4$ | CH | A/B = 70 : 30 | 90- 92 |

Unter den Verbindungen der Formel I sind diejenigen bevorzugt, in denen A und B 3-Chlorphenyl, 4-Bromphenyl, 3-Trifluormethylphenyl, 2,6-Dichlorphenyl, 4-tert.-Butylphenyl und insbesondere Phenyl, 2-Chlorphenyl, 4-Chlorphenyl sowie 2,4-Dichlorphenyl bedeuten.

Zur Herstellung antiviraler Mittel eignen sich sowohl die einheitlichen Diastereomeren bzw. Enantiomeren wie auch deren Gemische.

Als übliche Säuren zur Bildung physiologisch verträglicher Salze kommen vorzugsweise Halogenwasserstoffsäuren, wie z.B. Chlorwasserstoffsäure und Bromwassrstoffsäure, insbesondere die Chlorwasserstoffsäure, mit der die erfindungsgemäßen Verbindungen besonders gut kristallisierende Salze bilden, in Frage, ferner Phosphorsäure, Salpetersäure, Schwefelsäure, mono- und bifunktionelle Carbonsäuren und Hydroxycarbonsäuren, wie Essigsäure, Oxalsäure, Maleinsäure, Bernsteinsäure, Fumarsäure, Weinsäure, Zitronensäure, Salicylsäure, Sorbinsäure, Milchsäure sowie Sulfonsäuren wie p-Toluolsulfonsäure und 1,5-Naphthalindisulfonsäure.

Die Verbindungen lassen sich wie in der DE-OS 32 18 129 bzw. 32 18 130 beschrieben herstellen.

So kann man eine Verbindung der Formel II

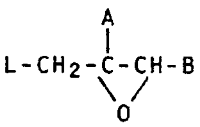

(II),

in welcher A und B die angegebenen Bedeutungen haben und L eine nukleophil substituierbare Abgangsgruppe darstellt, mit einer Verbindung der Formel III

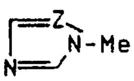

(III),

in der Me ein Wasserstoffatom oder ein Metallatom bedeutet und Z für eine CH-Gruppe oder ein Stickstoffatom steht, zur Umsetzung bringen.

Die Reaktion erfolgt - falls Me ein Wasserstoffatom bedeutet - gegebenenfalls in Gegenwart eines Lösungs- oder Verdünnungsmittels, gegebenenfalls unter Zusatz einer anorganischen Base und gegebenenfalls unter Zusatz eines Reaktionsbeschleunigers bei Temperaturen zwischen 10 und 120° C. Zu den bevorzugten Lösungs- oder Verdünnungsmitteln gehören Ketone wie Aceton, Methylethylketon oder Cyclohexanon, Nitrile wie Acetonitril, Ester wie Essigsäureethylester, Ether wie Diethylether, Tetrahydrofuran oder Dioxan, Sulfoxide wie Dimethylsulfoxid, Amide wie Dimethylformamid, Dimethylacetamid oder N-Methylpyrrolidon, ferner Sulfolan oder entsprechende Gemische.

Geeignete Basen, die gegebenenfalls auch als säurebindende Mittel bei der Reaktion verwendet werden können, sind beispielsweise Alkalihydroxide wie Lithium-, Natrium-oder Kaliumhydroxid; Alkalicarbonate wie Natrium- oder Kaliumcarbonat oder Natrium- und Kaliumhydrogencarbonat, Überschüsse an 1,2,4-Triazol, Pyridin oder 4-Dimethylaminopyridin. Es können aber auch andere übliche Basen verwendet werden.

Als Reaktionsbeschleuniger kommen vorzugsweise Metallhalogenide wie Natriumiodid oder Kaliumiodid, quarternäre Ammoniumsalze wie Tetrabutylammoniumchlorid, -bromid oder -iodid, Benzyl-triethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150° C, drucklos oder unter Druck, kontinuierlich oder diskontinuierlich durchgeführt.

Ist Me ein Metallatom wird die Reaktion gegebenenfalls in Gegenwart eines Lösungs- oder Verdünnungsmittels und gegebenenfalls unter Zusatz einer starken anorganischen oder organischen Base bei Temperaturen zwischen -10 und 120° C durchgeführt. Zu den bevorzugten Lösungs- oder Verdünnungsmitteln gehören Amide wie Dimethylformamid, Diethylformamid, Dimethylacetamid, Diethylacetamid, N-Methylpyrrolidon, Hexamethyl-phosportriamid, Sulfoxide wie Dimethylsulfoxid und schließlich Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalin-lithium, -natrium oder -kalium.

Die so erhaltenen Verbindungen der Formel I werden nach üblichen Methoden isoliert, gegebenenfalls gereinigt und gegebenenfalls mit physiologisch verträglichen Säuren in ihre Salze überführt.

Die Verbindungen der Formel I zeigen eine starke antivirale Aktivität, speziell gegen Herpes-Viren, wie folgende Beispiele zeigen (als Vergleichsverbindung diente Vibunazol):

Beispiel A

Zellkulturversuche

Herpes Simplex Virus (HSV) und menschliches Cytomegalovirus lassen sich in Kulturen von Affennierenzellen gut vermehren. Die Infektionen sind durch ca. 8 bis 12 h nach der Infektion auftretende charakteristische, mikroskopisch gut zu beobachtende und zu beurteilende cytopathische Effekte (CPE) gekennzeichnet. Antivirale Eigenschaften von Testsubstanzen, die für die Wirtszellen nicht toxisch sind bzw. ihre Wirkung gegen die infizierenden Viren in für die Zellen nicht toxischer Konzentration entfalten, können anhand der Reduktion des cytopathischen Effekts in diesem System gut charakterisiert werden.

Verwendet wurden Affennieren-zellen des Stammes RC-37 Rita (Italodiagnostics, Rom), die in basalem Eagle's Medium (BME; Eagle, H., J. Exp. Med. 102, 595-601, 1955) bis zur Infektion mit den HSV-1 Stämmen ANG (Schröder et al., Intervirology 6, 270-284, 1976) und WAL (Kirchner et al., J. Immunol. 117, 1753, 1978 ) gezüchtet wurden. Bei der Virusinfektion wurde das BME Medium gegen Medium 199 (Morgan et al., Proc. Soc. Exp. Biol., Med. 73, 1-8, 1950) ausgetauscht. Infiziert wurden die Zellen nach Ausbildung eines fast-konfluenten Rasens mit einer Multiplizität von zwei infektiösen Viruspartikeln [= 2 PFU (= plaque forming units)] pro Zelle. Die Virusstammsuspensionen hatten Titer zwischen $2 \times 10^7$ und $1 \times 10^8$ PFU/ml. Die Testsubstanzen wurden zu verschiedenen Zeiten zwischen 1 h vor bis 2 h nach der Infektion in nicht cytotoxischer Konzentration in das Medium der infizierten Zellen gegeben. Die Cytotoxizitätsgrenzen wurden anhand von Wachstumskurven und Koloniebildungsvermögen vereinzelter Zellen getestet. Die Wirkung der Substanzen wurde gegenüber von Kontrollkulturen sowohl anhand mikroskopischer Beobachtung als auch durch Ermittlung der Zellnachkommenviren verfolgt. Die Virustiter wurden nach Russel (Nature, London, 195, 1028-1029, 1962) bestimmt.

Hierbei zeigten die Verbindungen der vorliegenden Erfindung eine deutlich bessere Hemmung des CPE als Vibunazol. Auch die ermittelten Virus-Titer waren nach Applikationen der Verbindungen I, insbesondere der Substanz Nr. 3 deutlich niedriger als im Falle der Vergleichsverbindung.

Beispiel B

Test antiviraler Wirksamkeit im Maus-Modell der HSV-Infektion

Die experimentelle HSV-Infektion von suszeptiblen Inzucht-Mäusestämmen ist ein heute gut etabliertes, allgemein benutztes Versuchssystem für Pathogenitätsstudien von Herpesviren (Zisman et al., J. Immunol. 104, 1155-1159, 1970; Stevens and Cook, J. Epth. Med. 133, 19-38, 1971; Lopez, Nature London, 258, 152-153, 1957; Kirchner et al., Zeitsch. F. Immunitätsforsch. und exp. Therapie, 154, 147-154, 1978; Kaerner et al., J. Virol., 46, 83-93, 1983). Nach peripherer (i.p.) Infektion von Mäusen mit HSV-1 breitet sich die Infektion zunächst in der Milz aus, später (3 bis 4 Tage) nach Infektion befällt das Virus das ZNS der Tiere, gelangt ins Gehirn und tötet die Mäuse ca. 8 bis 10 Tage nach der Infektion durch Enzephalitis. Die i.p.-Infektion hat gegenüber anderen peripheren Infektionsarten (z.B. intravaginal etc.) Den Vorteil der genauen Dosierbarkeit.

Bei den vorliegenden Versuchen wurden Mäuse mit $10^6$ PFU HSV-1 WAL pro Tier i.p. infiziert. Die zu testenden Substanzen wurden den infizierten Tieren zu verschiedenen Zeiten nach der Infektion ebenfalls i.p. appliziert. Der Krankheitsverlauf wurde registriert. Bei allen Mäusen (getöteten und überlebenden) wurden die Gehirne auf die Präsenz von infektiösem Virus untersucht.

Die Applikation von Verbindungen der vorliegenden Erfindung - beispielsweise 3 - bewirkten eine gegenüber den Kontrollen signifikante Abnahme der Letalitätsrate. Die Vergleichsverbindung zeigte hier keine Wirkung.

Beispiel C

Test antiviraler Wirkung im Meerschweinchen-cutan Infektionsmodell

Diese Versuche wurden im wesentlichen nach dem Protokoll von Huber et al. (J. Invest. Dermatol. 62, 92-95, 1974) durchgeführt.

400 bis 500 g schwere Meerschweinchen wurden im Bereich der Hinterflanke enthaart, narkotisiert (Nembutal) und mit einem multiplen Nadelschußapparat (nach Buntschied) mit HSV-1 WAL (ca. $1 \times 10^5$ bis $5 \times 10^5$ PFU/Tier) intracutan infiziert. Die zu testenden Substanzen wurden peroral zu verschiedenen Zeiten (2 bis 48 h) nach der Infektion mit der Schlundsonde appliziert. Die Ausbildung und das Abheilen von viral-bedingten Läsionen an den Infektionsstellen wurden verfolgt und fotographisch dokumentiert.

Während die Applikation der Vergleichsverbindung einen gegenüber unbehandelten Kontrollen statistisch nicht signifikanten Einfluß auf den Infektionsverlauf zeigte, bewirkten die Verbindungen der vorliegenden Erfindung - beispielsweise gemäß Verbindung 3 - in derselben Dosierungsmenge eine geringere Ausbildung sowie ein rascheres Abheilen dieser viral-bedingten Läsionen.

Die Verbindungen der Formel I sind daher besonders geeignet zur enteralen, parenteralen und äußerlichen Behandlung von Virusinfektionen an Mensch und Tier and stellen somit eine wertvolle Bereicherung der Therapiemöglichkeiten dar. Folgende Indikationen seien speziell für die Anwendung am Menschen gennant: Herpes labialis, Herpes genitalis, Herpes Zoster (Gürtelrose), Varizellen (Windpocken), Keratoconjunctivitis herpetica, infektiöse Mononucleose und Cytomegalovirus-Infektionen. In der Tiermedizin kommen insbesondere Pseudorabies-Virus-Infektionen bei Schwein und Rind, Rhinotracheritis-Virus-Infektionen des Rindes, Rhinopneumonitis-Virus-Infektionen des Pferdes sowie die Marek'sche Kranheit bei Hühnern als Anwendungsgebiete in Betracht.

Die Verbindungen können allein oder zusammen mit anderen bekannten Wirkstoffen, insbesondere Antibiotika, verwendet werden.

Die Herstellung der chemotherapeutischen Mittel oder Zubereitungen mit üblichen festen, halbfesten oder flüssigen Trägerstoffen oder Verdünnungsmitteln und den üblicherweise verwendeten pharmazeutisch-technischen Hilfsstoffen entsprechend der gewünschten Applikationsart mit einer zur Anwendung geeigneten Dosierung erfolgt in üblicher Weise, insbesondere durch Vermischen (vgl. H Sucker et al, Pharmazeutische Technologie, Thieme-Verlag, Stuttgart, 1978).

Als Darreichungsformen kommen beispielsweise Tabletten, Dragees, Kapseln, Pillen, Suppositorien, wäßrige Lösungen, Suspensionen und Emulsionen, gegebenenfalls sterile injizierbare Lösungen, nichtwäßrige Emulsionen, Suspensionen und Lösungen, Salben, Cremes, Pasten und Lotions in Betracht.

Die therapeutisch wirksame Verbindung ist in pharmazeutischen Zubereitungen vorzugsweise in einer Konzentration von 0,5 bis 90 Gew.% der Gesamtmischung vorhanden.

Im allgemeinen können bei oraler Verabfolgung sowohl in der Human- als auch in der Veterinärmedizin der oder die Wirkstoffe in Mengen von 0,1 bis etwa 10,0, vorzugsweise 0,2 bis 6 mg/kg Körpergewicht pro Tag, vorzugsweise in Form mehrerer Einzelgaben zur Erzielung der gewünschten Ergebnisse verabreicht werden. Es kann jedoch erforderlich sein, von den genannten Dosierungen abzuweichen, und zwar in Abhängigkeit von der Art und der Schwere der Erkrankung, der Art der Zubereitung und der Applikation des Arzeimittels, sowie dem Zeitraum bzw. Intervall, innerhalb welchem die Verabreichung erfolgt. So kann es in einigen Fallen ausreichend sein, mit weniger als der obengenannten Menge Wirkstoffe auszukommen, während in anderen Fällen die oben angeführte Wirkstoffmenge überschritten werden muß.

Beispiele für pharmazeutische Zubereitungen:

<u>Beispiel I</u>

Tablette mit 250 mg Wirkstoff

Zusammensetzung für 1.000 Tabletten:

| | |
|---|---|
| Wirkstoff Nr. 3 | 250 g |
| Kartoffelstärke | 100 g |
| Milchzucker | 50 g |
| Gelatinelösung 4 % | 45 g |
| Talkum | 10 g |

Herstellung:

Der fein gepulverte Wirkstoff, Kartoffelstärke und Milchzucker werden gemischt. Die Mischung wird mit der Gelatinelösung durchfeuchtet, feinkörnig granuliert und getrocknet. Das trockene Granulat wird gesiebt, mit Talkum vermischt und auf einer Rundläufer-Tablettiermaschine zu Tabletten verpreßt. Die Tabletten werden in dicht schließende Behälter aus Polypropylen gefüllt.

Beispiel II

Creme mit 1 % Wirkstoff

| | |
|---|---|
| Wirkstoff Nr. 3 | 1,0 g |
| Glycerinmonostearat | 10,0 g |
| Cetylalkohol | 4,0 g |
| Polyethylenglykol-400-stearat | 10,0 g |
| Polyethylenglykol-sorbitanmonostearat | 10,0 g |
| Propylenglykol | 6,0 g |
| p-Hydroxybenzoesäuremethylester | 0,2 g |
| Entmineralisiertes Wasser | ad 100,0 g |

Herstellung:

Der feinst gepulverte Wirkstoff wird mit Propylenglykol suspendiert und die Suspension in die auf 65° C erwärmte Schmelze aus Glycerinmonostearat, Cetylalkohol, Polyethylenglykol-400-stearat und Polyethylenglykolsorbitanmonostearat gerührt. In diese Mischung wird die 70° C heiße Lösung des p-Hydroxybenzoesäuremethylesters in Wasser emulgiert. Nach dem Erkalten wird die Creme über eine Kolloidmühle homogenisiert und in Tuben abgefüllt.

Beispiel III

Puder mit 1 % Wirkstoff

| | |
|---|---|
| Wirkstoff Nr. 3 | 1,0 g |
| Zinkoxid | 10,0 g |
| Magnesiumoxid | 10,0 g |
| Hochdisperses Siliciumdioxid | 2,5 g |
| Magnesiumstearat | 1,0 g |
| Talkum | 75,5 g |

Herstellung:

Der Wirkstoff wird auf einer Luftstrahlmühle mikronisiert und mit den anderen Bestandteilen homogen vermischt. Die Mischung wird durch ein Sieb (Maschenweite Nr.7) geschlagen und in Polyethylenbehälter mit Streueinsatz abgefüllt.

Die folgenden Beispiele erläutern Herstellung der Verbindungen der Formel I.

Herstellung der Ausgangsstoffe

Beispiel a

In eine Lösung von 229 g 2,4-Dichlorbenzyltriphenylphoshoniumchlorid und 800 ml trockenem Methanol wurden bei 10° C 63,6 g Kalium-tert.-butylat in 300 ml trockenem Methanol eingetragen und nach 0,5 h 77,2 g 4-Chloracetophenon zugegeben. Die Reaktionslösung wurde 3 h unter Rückfluß gekocht, dann bei Raumtemperatur das abgeschiedene Salz abfiltriert und das Filtrat im Vakuum eingedampft. Durch Dispergieren des Rückstandes mit Petrolether (50 bis 70° C) wurde vom Triphenylphosphinoxid abgetrennt und die Lösung im Vakuum eingedampft. Der Rückstand wurde in 1 l Tetrachlorkohlenstoff aufgenommen und mit 81,7 g N-Bromsuccinimid und 4 g 2,2-Azo-isobuttersäuredinitril unter Rückfluß gekocht. Nach beendeter Reaktion wurde das Succinimid durch Filtration abgetrennt, das Filtrat im Vakuum eingedampft und der Rückstand aus Methanol umkristallisiert. Man erhielt 73,4 g (38,8%) Z-1-(2,4-Dichlorphenyl)-2(4-chlorphenyl)-3-brom-propen-1, Fp. = 128° C.

Beispiel b

58,9 g Z-1-(2,4-Dichlorphenyl)-2-(4-chlorphenyl)-3-brom-propen-1 (vgl. Beispiel a) wurden mit 52,3 g 3-Chlorperoxybenzoesäure in 590 ml Chloroform unter Rückfluß gekocht. Nach beendeter Reaktion wurde die Chloroform-Phase mit wäßriger Natriumhydrogencarbonat-Lösung und Wasser säurefrei gewaschen, über Natriumsulfat getrocknet und im Vakuum eingedampft. Aus dem Rückstand erhielt man mit Methanol zwei Kristallfraktionen:

1. 41,3 g (70,2 %) 2-Brommethyl-2-(4-chlorphenyl)-3-(2,4-dichlorphenyloxiran (Isomer A), Fp. = 98 bis 99 °C

und

2. 12 g (20,4 %) 2-Brommethyl-2-(4-chlorphenyl)-3-(2,4-dichlorphenyl)-oxiran (Isomer B), Fp. = 93 bis 95 °C.

Herstellung der Endprodukte

Beispiel c

Eine Lösung von 10 g 2-Brommethyl-2-(4-chlorphenyl)-3-(2,4-dichlorphenyl)-oxiran (Isomer A) (vgl. Beispiel b) in 50 ml N,N-Dimethylformamid wurde bei 100 °C zu einer Schmelze aus 15,6 g Imidazol mit 1,37 g Natriummethylat, aus der man das freigesetzte Methanol zuvor abdestillieren ließ, zugetropft. Nach 8 h wurde die Reaktionslösung auf Wasser gegeben und mit Essigsäureethylester extrahiert; die organische Phase wurde mit Wasser gewaschen, über Natriumsulfat getrocknet und im Vakuum eingedampft. Der Rückstand wurde an einer Kieselgelsäule mit Methylenchlorid/Methanol (100:2) chromatographiert. Die gereinigten Fraktionen wurden eingedampft und aus Diisopropylether kristallisiert. Man erhielt 4,6 g (47,5 %) 2-(1H-Imidazol-1-yl-methyl)-2-(4-chlorphenyl)-3-(2, 4-dichlorphenyl)-oxiran (Isomer A), Fp. = 102 bis 103 °C.

Entsprechend Beispiel a bis c) lassen sich die auf den Seiten 2 bis 5 angeführten Verbindungen herstellen.

## Patentansprüche

**1.** Verwendung der Azolylmethyloxirane der Formel I

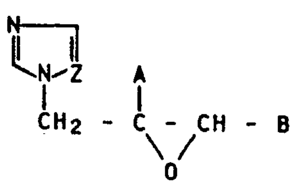

(I),

in welcher
A und B gleich oder verschieden sind und unabhängig voneinander Alkyl mit 1 bis 4 Kohlenstoffatomen, Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen, Phenoxy sowie den Phenylsulfonylrest substituiert sein kann, und
Z für eine CH-Gruppe oder ein Stickstoffatom steht, sowie deren physiologisch verträgliche Salze zur Herstellung von Arzneimitteln mit antiviraler Wirksamkeit.

**2.** Verwendung der Azolylmethyloxirane der Formel I gemäß Anspruch 1, in welcher A und B gleich oder verschieden sind und unabhängig voneinander Naphthyl, Biphenyl oder Phenyl bedeuten, wobei der Phenylrest durch Halogen, Nitro, Alkyl, Alkoxy oder Halogenalkyl mit jeweils 1 bis 4 Kohlenstoffatomen sowie der Halogenalkyl mit jeweils 1 bis 4 Kohlenstoffatomen sowie der Phenoxyrest substituiert sein kann, und Z für die CH-Gruppe oder ein Stickstoffatom steht sowie deren physiologisch verträgliche Salze zur Herstellung von Arzneimitteln mit antiviraler Wirksamkeit.

**3.** Verwendung der Azolylmethyloxirane der Formel I gemäß Anspruch 1, in der A und B gleich oder verschieden sind und unabhängig voneinander Phenylreste bedeuten, die durch Halogen substituiert

sein können und Z die in Anspruch 1 angegebene Bedeutung hat, sowie deren physiologisch verträgliche Salze zur Herstellung von Arzneimitteln mit antiviraler Wirksamkeit.

## Claims

1. Use of an azolylmethyloxirane of the formula I

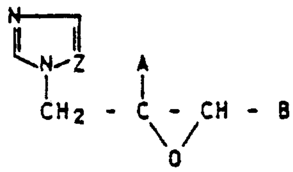

(I)

where A and B are identical or different and independently of one another are each alkyl of 1 to 4 carbon atoms, naphthyl, biphenyl or phenyl, and the phenyl radical may be substituted by halogen, nitro, alkyl, alkoxy or haloalkyl, each of 1 to 4 carbon atoms, phenoxy or phenylsulfonyl, and Z is a CH group or nitrogen, or its physiologically tolerated salts for the preparation of drugs possessing antiviral activity.

2. Use of an azolylmethyloxirane of the formula I as claimed in claim 1, wherein A and B are identical or different and independently of one another are each naphthyl, biphenyl or phenyl, and the phenyl radical may be substituted by halogen, nitro, alkyl, alkoxy or haloalkyl, each of 1 to 4 carbon atoms, or phenoxy, and Z is the CH group or nitrogen, or its physiologically tolerated salts for the preparation of drugs possessing antiviral activity.

3. Use of an azolylmethyloxirane of the formula I as claimed in claim 1, wherein A and B are identical or different and independently of one another are each phenyl which may be substituted by halogen, and Z has the meaning stated in claim 1, or its physiologically tolerated salts for the preparation of drugs possessing antiviral activity.

## Revendications

1. Utilisation des azolylméthyloxiranes de formule I

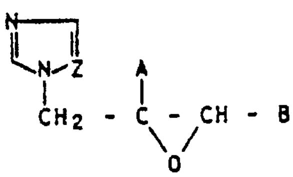

(I)

dans laquelle
A et B sont identiques ou différents et représentent, indépendamment l'un de l'autre, alkyle ayant 1 à 4 atomes de carbonne, naphtyle, biphényle ou phényle, le reste phényle pouvant être substitué par halogène, nitro, alkyle, alcoxy ou halogéno-alkyle (dans chaque cas à 1 à 4 atomes de carbone), par phénoxy, ainsi que par le reste phénylsulfonyle, et

Z représentant un groupe CH ou un atome d'azote, ainsi que leurs sels physiologiquement tolérés pour la préparation de médicaments à effet antiviral.

2. Utilisation des azolylméthyloxiranes de formule I, selon la revendication 1, dans laquelle A et B sont identiques ou différents et représentent, indépendamment l'un de l'autre, naphtyle, biphényle ou phényle, le reste phényle pouvant être substitué par halogène, nitro, alkyle, alcoxy ou halogénoalkyle, dans chaque cas à 1 à 4 atomes de carbone, ainsi que par le reste phénoxy, et Z est mis pour le groupe CH ou un atome d'azote, ainsi que leurs sels physiologiquement tolérés, pour la préparation de

médicaments à effet antiviral.

3. Utilisation des azolylméthyloxiranes de formule I, selon la revendication 1, dans laquelle A et B sont identiques ou différents et représentent, indépendamment l'un de l'autre, des restes phényle pouvant être substitués par halogène, et Z à la signification donnée dans la revendication 1, ainsi que leurs sels physiologiquement tolérés, pour la préparation de médicaments à effet antiviral.